# EUROPEAN PATENT APPLICATION

(11) **EP 4 745 981 A1**
(43) Date of publication of application: **20.05.2026**
(21) Application number: 24306917.6
(22) Date of filing: 15.11.2024
(51) Int. Cl.: G16H 20/40, A61B 6/03, A61B 6/00, A61C 13/00, G06T 19/00, G16H 30/40, G16H 40/63, G16H 40/67, G16H 50/70

(54) **METHOD AND SYSTEM FOR ASSISTING A PRACTITIONER IN PLANNING DENTAL IMPLANTS**

(71) Applicant: TROPHY SAS, 77435 Marne la Vallee Cedex 2 (FR); Carestream Dental LLC, Atlanta, GA 30339 (US)
(72) Inventor: JACOB, Jean-Pascal, 77435 MARNE-LA-VALLEE CEDEX 2 (FR); PETILLO, Julien, 77435 MARNE-LA-VALLEE CEDEX 2 (FR); OUDAHMANE, Lylia, 77435 MARNE-LA-VALLEE CEDEX 2 (FR)
(74) Representative: Casalonga

(57) **Abstract**

According to some embodiments of the disclosure, it is provided a computer-assisted method for assisting a practitioner for planning a dental implant, the method comprising obtaining (200, 205) a 3D representation of at least a portion of a dental arch, segmenting (235), from the 3D representation, teeth adjacent to a site of the dental implant and of an associated crown and segmenting (250), from the 3D representation, a bone comprising a portion of the site, selecting the dental implant and determining (260) a position of the dental implant as a function of the segmented teeth and bone, and displaying (265) at least a portion of the segmented teeth and bone and displaying the selected dental implant at the determined position.

## Description

### FIELD OF THE DISCLOSURE

The present disclosure relates to the field of implant planning. Particularly, but not exclusively, the disclosure relates to a method and system for assisting a practitioner in planning dental implants.

### BACKGROUND OF THE DISCLOSURE

Planning the implantation of a dental implant is generally required to choose the right dental implant (e.g., the dental implant having the correct dimensions), an optimum implant insertion axis (i.e., drilling axis), and the right crown prothesis.

To that end, a practitioner needs to have relevant information, in particular a precise understanding of the intended implant area, that is to say the bone and the surrounding anatomical obstacles (mandibular nerve canal, other implants or teeth, sinus floor, etc.). The most suitable imaging exam for that task is based on the Cone Beam Computed Tomography (CBCT) technology, because it is a non-invasive and fully-3D technology that emphasizes the bone and teeth materials within the field of view.

Once a dental implant and a crown prothesis have been selected and an implant insertion axis has been identified, an adapted surgical guide may be determined. It may be determined using a stone model of an arch, obtained from data resulting from a CBCT scan of the arch, or using data obtained by an intra-oral scan of the teeth (e.g., by an optical scan), from which a 3D mesh may be obtained. Since most of the surgical guides are anchored or positioned according to the crowns of the teeth (i.e., to the visible parts of the teeth), an intra-oral scan of the teeth, providing precise reference points, is commonly performed prior to implant planning, at least for determining a surgical guide.

Typically, planning a dental implant is carried out in two successive steps:
- positioning a temporary or virtual dental crown, also referred to as waxup and
- positioning the dental implant as a function of the waxup, the bone, and anatomical obstacles.

Since there is a strong mechanical dependency between the dental crown and the dental implant, the axes of the dental crown and of the dental implant must be close enough to handle properly the occlusal forces (i.e., they should be sufficiently similar (approximately parallel and aligned) to properly handle occlusal forces). As a consequence, the two above mentioned steps should be carried out in the given order. In other words, the waxup aesthetic and functional position strongly guides the implant positioning and, for that reason, implant planning should first be directed to the positioning of the temporary or virtual dental crown and then that of the dental implant.

While a dental implant planning may be carried out with a stone model of an arch and a temporary dental crown, it may instead be carried out virtually on a computer. However, such a digital dental implant planning is a tedious task since the practitioner must be able to correctly position a virtual dental crown, and then a dental implant, by navigating into opacity-dependent 3D views on a 2D screen and independently manipulating multiple 3D objects on a 2D screen, which is done by switching/scrolling between slices and/or rotating several times the 3D view while adjusting contrast and opacity, so as to check multiple different clinical margins, permissible relative orientation and alignment of a crown and an implant, and the like.

Therefore, there is a need for assisting a practitioner in planning dental implants using digital tools, to improve the positioning of the dental implant and reduce the costs of preparing dental implant operations.

### SUMMARY OF THE DISCLOSURE

The present disclosure has been devised to address one or more of the foregoing concerns.

In this context, there is provided a method, a system, and a non-transitory computer-readable storage medium for assisting a practitioner in planning dental implants.

According to an aspect of the disclosure, there is provided a computer-assisted method for assisting a practitioner for planning a dental implant, the method comprising:
obtaining a 3D representation of at least a portion of a dental arch;
segmenting, from the 3D representation, teeth adjacent to a site of the dental implant and an associated crown, and segmenting, from the 3D representation, a bone comprising a portion of the site;
selecting the dental implant and determining a position of the dental implant as a function of the segmented teeth and bone; and
displaying at least a portion of the segmented teeth and bone and displaying the selected dental implant at the determined position.

The method according to the disclosure makes it possible to save time to the practitioner by limiting the interactions involving the practitioner and by displaying directly the dental implant at a correct position, without having to search for this position, according to surrounding teeth and anatomical structures.

According to particular embodiments, the method further comprises segmenting, from the 3D representation, anatomical features in the site and/or in the neighborhood of the site, the selecting the dental implant and the determining the position of the dental implant being further carried out as a function of the segmented anatomical features.

Still according to particular embodiments, the 3D representation comprises at least Cone Beam Computed Tomography (CBCT), 3D volume data, the segmenting the bone comprising a portion of the site comprising segmenting the CBCT 3D volume data.

Still according to particular embodiments, the 3D representation further comprises at least one 3D mesh model registered with the CBCT 3D volume data, the segmenting the teeth adjacent to the site comprising segmenting the 3D mesh model. The method may further comprise registering the at least one 3D mesh model with the CBCT 3D volume data.

Still according to particular embodiments, the method further comprises positioning the associated crown in the site, between the segmented teeth, the dental implant being selected as a function of the segmented teeth, the segmented bone, and the positioned associated crown.

Still according to particular embodiments, the method further comprises determining a shape of at least a portion of dental arch, the associated crown being placed as a function of the determined shape and of a position of the segmented teeth relative to the determined shape.

Still according to particular embodiments, the method further comprises displaying the associated crown.

Still according to particular embodiments, the position of the dental implant is determined as a function of the segmented teeth, the segmented bone, and the positioned associated crown.

Still according to particular embodiments, the method further comprises selecting the associated crown.

Still according to particular embodiments, the method further comprises obtaining a dental implant type, the dental implant being selected as a function of the segmented teeth, the segmented bone, and the dental implant type.

Still according to particular embodiments, selecting the dental implant and determining a position of the dental implant comprise determining a plurality of possible positions for the dental implant and selecting at least one dental implant for at least one position of the plurality of possible positions.

Still according to particular embodiments, selecting the dental implant comprises determining features of the dental implant, the dental implant being selected as a function of the determined features.

Still according to particular embodiments, displaying a least a portion of the segmented teeth and bone and displaying the selected dental implant at the determined position comprises displaying a least a portion of the segmented teeth and bone and displaying the selected dental implant at the determined position in a plurality of views.

Still according to particular embodiments, the plurality of views comprises at least one cross-sectional view according to a plan perpendicular to a Mesio-Distal axis and comprising at least a portion of the dental implant and comprises at least one cross-sectional view according to a plan perpendicular to a Vestibulo-Lingual axis and comprising at least a portion of the dental implant.

Still according to particular embodiments, the method further comprises displaying additional data, the additional data comprising at least one of a distance from the implant to an external surface of the bone, a distance from the implant to an anatomical feature in the site and/or in the neighborhood of the site, an angle of the implant with respect to a predetermined frame reference, an arch path, and/or representations of one or more anatomical features.

Still according to particular embodiments, the method further comprises obtaining an indication of a change of the position of the dental implant and updating display of the selected dental implant according to the changed position.

According to other aspects of the disclosure, there is provided a device comprising a processing unit configured for carrying out each step of the method described above. The other aspects of the present disclosure have advantages similar to the one above-mentioned aspect.

At least parts of the methods according to the disclosure may be computer implemented. Accordingly, the present disclosure may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present disclosure may take the form of a non-transitory computer-readable storage medium having computer executable program code for conducting the method(s) embodied in the medium. A non-transitory computer-readable storage medium may comprise a storage medium such as a floppy disk, a CD-ROM, a hard disk drive, a magnetic tape device or a solid-state memory device and the like. The present disclosure may also take the form of a computer program product for a programmable apparatus, the computer program product comprising a sequence of instructions for implementing each of the steps of the method described above when loaded into and executed by the programmable apparatus.

### BRIEF DESCRIPTION OF THE DRAWINGS

Embodiments of the disclosure will now be described, by way of example only, and with reference to the following drawings in which:
Figure 1a is a perspective representation of a portion of a dental arch comprising a site of a dental implant and of an associated crown;
Figure 1b is a perspective representation of a portion of a dental arch illustrating a frame reference to be used for locating points in space;
Figure 2 illustrates an example of steps of a method according to some embodiments of the disclosure for assisting a practitioner in planning dental implants;
Figure 3 illustrates a 3D mesh model of a tooth, used to determine the main axis of the tooth;
Figures 4a, 4b, and 4c illustrate an example of a method for determining the position and orientation of a virtual crown;
Figures 5a to 5d illustrate an example of a method for determining a set of possible positions for an implant;
Figure 6 illustrates an example of a method for selecting implants that have dimensions that comply with clinical margins;
Figure 7 illustrates an example of an implant to be used and of its position;
**Figure** 8 illustrates an example of a graphical user interface that may be used to display and manipulate an implant in an implant site, the implant being initially automatically selected and positioned; and
Figure 9 is a schematic block diagram of a computer for implementation of one or more embodiments of the disclosure.

### DETAILED DESCRIPTION OF THE DISCLOSURE

The following is a detailed description of particular embodiments of the disclosure, reference being made to the drawings in which the same reference numerals identify the same elements of structure in each of the Figures.

In the drawings and text that follow, like elements are designated with like reference numerals, and similar descriptions concerning elements and an arrangement or interaction of elements already described are omitted. Where they are used, the terms "first", "second", and so on, do not necessarily denote any ordinal or priority relation, but may simply be used to more clearly distinguish one element from another, unless specified otherwise.

In the context of the present disclosure, the terms "operator" and "user" are considered to be equivalent and refer to the practitioner, technician, or other person who acquires, views, and manipulates data represented as images, such as CBCT images and or 3D mesh model, on a display monitor. An "operator instruction" or "user instruction" is obtained from explicit commands entered by the operator or user, such as by using a computer mouse or by using a touch screen or a keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component. Depending on the context, the terms "implant" and/or "crown" may designate either a real implant, respectively a real crown, to be placed within the mouth of a patient, or a corresponding virtual implant, respectively a corresponding virtual crown, to be manipulated in a digital environment, for example through a Graphical User Interface (GUI), for example for planning purposes.

According to some embodiments of the disclosure, 3D data of a dental arch (or a portion of the dental arch) are acquired and processed to make it possible to select an implant and to determine a position and orientation of the implant, as a function of the implant site, the adjacent teeth, the anatomical features neighboring the implant site, etc. The selected implant may be displayed in a representation of the implant site, according to one or several 2D and/or 3D views, so that a practitioner may validate the position and orientation of the implant or adjust easily the position and/or orientation of the implant.

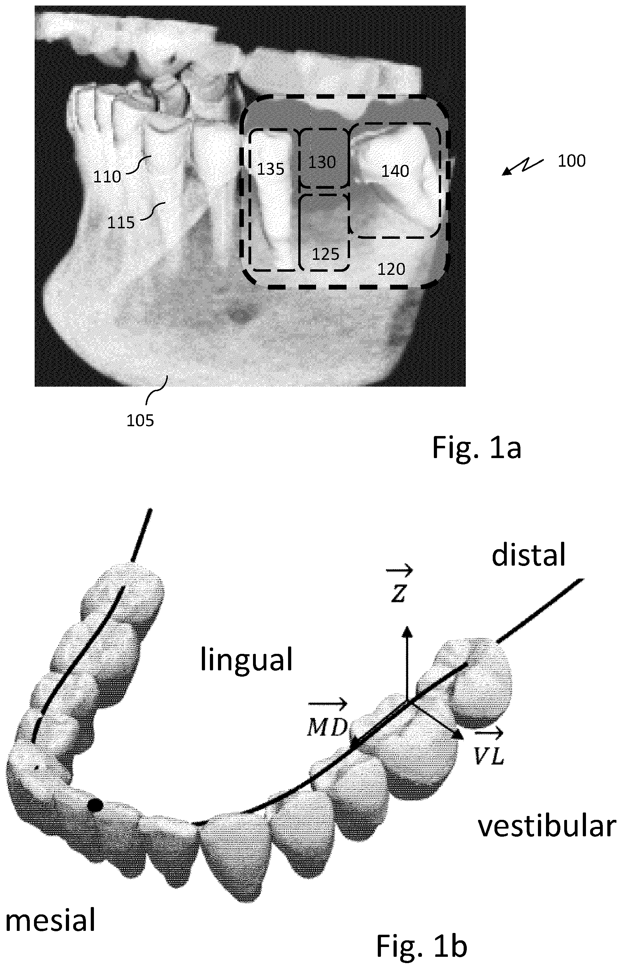

**Figure 1a** is a perspective representation of a portion 100 of a dental arch comprising a bone 105 and teeth, each tooth comprising a crown (e.g., crown 110) and a root (e.g., root 115). In addition, portion 100 of the dental arch comprises a site of a dental implant and of an associated crown, referred to as the implant site.

As illustrated, site 120 of the dental implant and of the associated crown comprises a portion of bone 125 where the dental implant is to be inserted and a space 130 located just above the portion of bone 125, wherein the crown is to be mounted. This space is located between neighboring teeth 135 and 140.

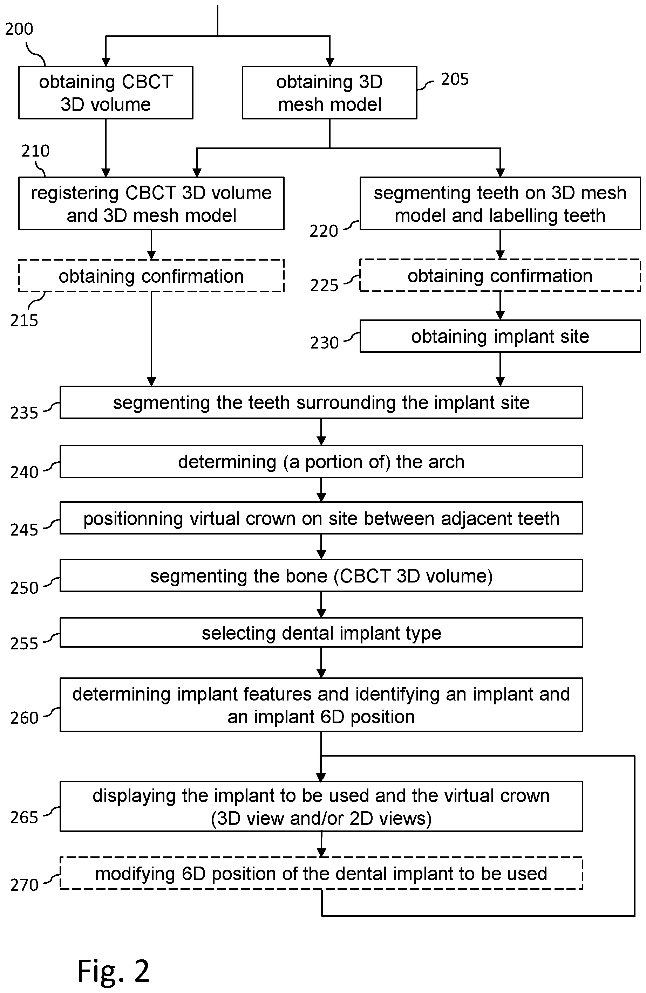

According to the example of Figure 1a, the representation of the portion of the dental arch is a combination of data obtained from a CBCT scanner and from data obtained from an intra-oral scanner, as described with reference to Figure 2.

In order to locate each point in space, in particular points belonging to bone 105, to a tooth, to a dental implant or to a crown, a reference frame may be associated with each tooth. According to a common practice, an orthonormal reference frame is used, in which the first axis is defined by the dental arch and oriented toward the mesial part of the arch, as illustrated with vector *̅M̅D̅*̅ in **Figure 1b**, the second axis is perpendicular to the first axis, belongs to an occlusal plane defined by the top point teeth of the considered dental arch, and oriented toward the vestibular part of the arch, as illustrated with vector *V̅L̅,* and the third axis is perpendicular to the first and the second axes and extends in the opposite direction of the teeth, as illustrated with vector *̅Z̅*̅.

**Figure 2** illustrates an example of steps of a method according to some embodiments of the disclosure for assisting a practitioner in planning dental implants.

As illustrated, first steps are directed to obtaining CBCT 3D volume data (step 200) and a 3D mesh model (step 205). The CBCT 3D volume data may be obtained from a standard CBCT x-ray device, for example the CBCT device marketed under the name CS9600 by Carestream Dental Inc., using a standard format, for example the dicom or mha format. The 3D mesh model may be obtained from a standard intraoral scanner, for example an optical intraoral scanner like the optical intraoral scanner marketed under the name CS 3800 by Carestream Dental Inc., using a standard format, for example the dicom or stl format. According to some embodiments, the CBCT 3D volume data comprise at least one jaw with a full arch. It may be used as a reference frame where the virtual crown and the virtual implant may be positioned and where the rightful areas and the anatomical obstacles may be depicted. Still according to some embodiments, the 3D mesh model is used to design surgical guides and to improve performance and precision, in particular in the crowns area. According to other embodiments, only CBCT 3D volume data are used.

Next, the CBCT 3D volume data and the 3D mesh model are registered (step 210), so that the same points of the CBCT 3D volume and of the 3D mesh model coincide once the CBCT 3D volume data and the 3D mesh model are registered. For the sake of illustration, registering the CBCT 3D volume data and the 3D mesh model may be based on thresholds in the CBCT 3D volume data and on contouring algorithms (e.g., marching cube algorithms) to deduce contour meshes in the CBCT 3D volume data making it possible to match corresponding features between meshes derived from the CBCT 3D volume data and the 3D mesh model. Still according to some embodiments, registration of the CBCT 3D volume and of the 3D mesh model may be improved by using ICP (Iterative Closest Points) like algorithms.

Once being registered, the CBCT 3D volume and the 3D mesh model form a merged 3D representation comprising reliable surface data and data characterizing the internal nature of the jaw.

As illustrated in Figure 2, registration of the CBCT 3D volume data and the 3D mesh model may be validated by the practitioner (step 215). To that end, one or more perspective views and/or one or more cross-sectional views of the registered CBCT 3D volume data and 3D mesh model may be displayed on a graphical user interface.

In parallel, before, or after registering the CBCT 3D volume data and the 3D mesh model, the teeth (or more precisely the crowns of the teeth) are segmented and labelled (step 220). Since the shape of the crowns is more precise on the 3D mesh model than on a 3D mesh derived from the CBCT 3D volume data, the crowns of the teeth (i.e., the coronal parts) are preferably segmented from the registered 3D mesh model (although the registered CBCT 3D volume data may also be used). The tooth labelling may be used for selecting the implant planning site on the merged 3D representation (or the registered CBCT 3D volume or the registered 3D mesh) and/or for selecting a virtual crown shape. In addition to labelling the teeth, the teeth segmentation may be used to optimize occlusion of the virtual crown with the antagonist tooth when positioning the virtual crown.

According to some embodiments, a deep learning (DL) algorithm may be used to segment the teeth on the registered 3D mesh, for example to depict one segmentation area per tooth and then, to carry out a precise tooth delimitation for each tooth. This may be done using a point cloud deep learning approach such as PointNet, a network developed by the Stanford University for 3D classification and segmentation.

As illustrated, since the teeth labelling should be accurate for a precise selection of the implant planning site, a user approval of the teeth labelling may be required (step 225).

Next, the implant site is obtained (step 230). For the sake of illustration, the implant site may be selected by the practitioner. It may be defined by the label of the missing tooth, where the implant is to be done. Obtaining the implant site provides information about the rough localization of the implant, as well as information about the virtual crown class (e.g. incisor, canine, premolar, or molar), making it possible to select one or more crowns in a tooth library. The obtained items of information about the implant site may also be used to guide the implant's dimensional choice strategy (e.g. thinner and longer in anterior sectors).

Next, the teeth surrounding the implant site (also referred to as the adjacent teeth) are segmented (step 235). According to some embodiments, segmenting the teeth surrounding the implant site starts by segmenting their crown from the 3D mesh model of the registered CBCT 3D volume data and 3D mesh model, and ends by segmenting their root from the registered CBCT 3D volume data. Still according to some embodiments, a 3D DL algorithm may be used to segment precisely an entire tooth from the registered CBCT 3D volume data. It is noted here that segmenting the teeth surrounding the implant site provides information on tooth implantation, in particular on tooth axis, which is useful for determining the proper axis of the virtual crown and/or of the virtual implant, as described herein below. It is also noted that the 3D DL algorithm may be trained with training data comprising, as input data, a set of voxels, such as a cube of voxels belonging to CBCT 3D volume data (e.g., a 3 centimetre cube), each set of voxels (or cube) being centred on a labelled tooth, and comprising, as output data, the segmented teeth of the set of voxels (or cube). The3D DL algorithm may be based on the 3D Unet.

There exist known algorithms that may be used for determining the axis of a tooth as a function of its shape. For the sake of illustration, the PCA (Principal Component Analysis) main axis may be first determined, which makes it possible to determine the crown side and the root side of the tooth. Next, the tooth axis may be refined by determining the center of the crown part on the occlusal side, and the center of the root part on the other (apexes) side, the refined axis joining both sides' centers.

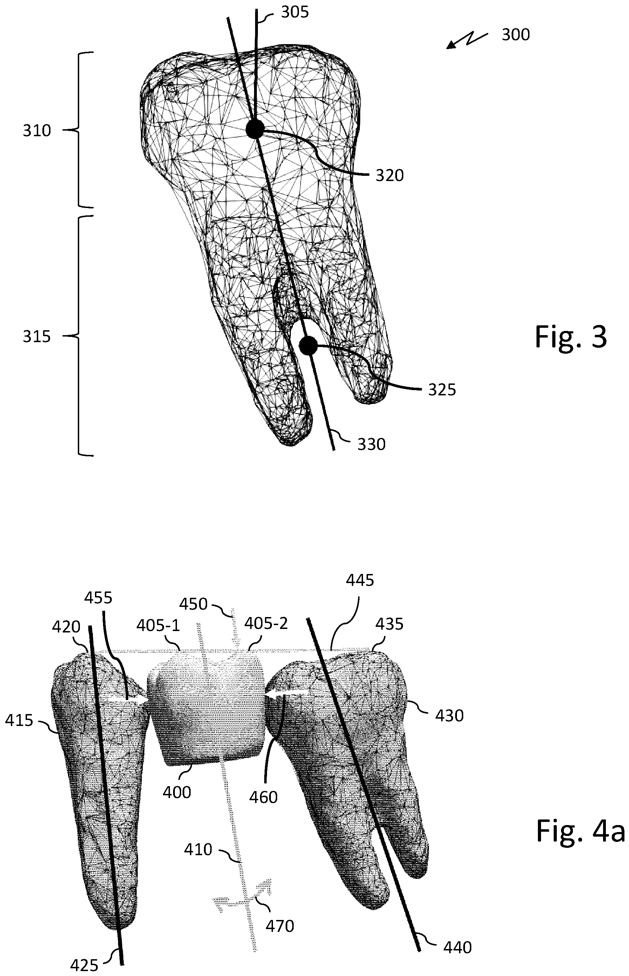

**Figure 3** illustrates a 3D mesh model of a tooth 300, used to determine the main axis of the tooth. After having determined the PCA main axis 305, the crown side 310 and the root side 315 are identified, making it possible to determine the center 320 of the crown part and the center 325 of the root part. The axis 330 of the tooth is then defined as the straight line passing through the two centers 320 and 325.

Turning back to Figure 2 and after having determined the axes of the teeth surrounding the implant site (or before or in parallel), the arch or a portion of the arch is determined (step 240), for example using the Al driven automatic panoramic curve mapping function, denoted "Automatic Arch", of the CS 3D Imaging software developed by Carestream Dental LLC, for example in its release 3.10.43 of January 15, 2024.

The determined arch may be a curved trace (or dental arch tracing) that follows the teeth and bone shape in an axial view, for example close to a level where the teeth are inserted in the bone, wherein the bone is drilled to insert the implant. The determined arch may be used to finely adjust the virtual crown positioning in a coherent alignment. According to some embodiments, a segmentation of the teeth and/or bone area may be used, making it possible to select a consistent level and to have a segmented shape to follow, on which to center the arch.

Next, a virtual crown is positioned in the implant site (step 245), between the adjacent teeth, depending on the determined arch and adjacent teeth axis. According to some embodiments, positioning is done according to 6 degrees of freedom corresponding to a position (3 values: MD (Mesio-Distal), VL (Vestibulo-Lingual) and Z (vertical or occlusal)) and an orientation (3 values: Tip (around VL), Torque (around MD) and Rotation (around Z)). In addition, a mesio-distal scale and/or a bucco-lingual scale and/or a vertical scale of the virtual crown may be done, for example to select a virtual crown (or to adapt the generic type of crown previously selected) and to fill precisely the empty space between the adjacent teeth.

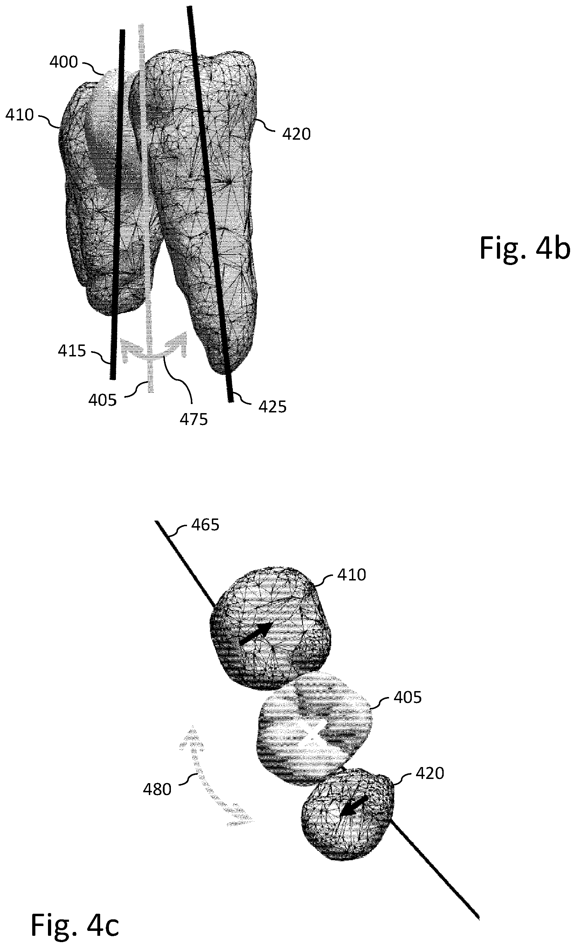

The three position values may be determined as a function of the adjacent teeth, for example by aligning the virtual crown with the crowns of the adjacent teeth and by centering the virtual crown with regard to the crowns of the adjacent teeth. Regarding the three orientation values, they may be determined as a function of the axes of the adjacent teeth. The shape of the arch, for example as represented by the dental arch tracing, may also be used to refine some orientation or position values (as illustrated in Figure 4c).

**Figures 4a****,** **4b,** and **4c** illustrate an example of a method for determining the position and orientation of a virtual crown.

As illustrated, virtual crown 400, comprising top points 405-1 and 405-2 and having axis 410, is placed in between adjacent tooth 415, comprising top point 420 and having axis 425, and adjacent tooth 430, comprising top point 435 and having axis 440.

As illustrated with line 445 and arrow 450, the vertical position of the virtual crown (i.e., its position according to the Z axis) may be set in such a way that its top points 405-1 and 405-2 are aligned with the top points 420 and 435 of the adjacent teeth. Regarding the MD axis, virtual crown 400 may be centered between teeth 415 and 430. Regarding the VL axis, virtual crown 400 may be aligned with teeth 415 and 430 along arch 465, i.e., the VL offset of virtual crown 400 may be set as an intermediate value between adjacent teeth offsets to arch 465.

As illustrated with arrows 455 and 460, the MD scale of virtual crown 400 may be determined as a function of the distance between teeth 415 and 430 along the arch. As illustrated with arrows 470 and 475, the tip and the torque may be determined as an intermediate value between the tip and the torque of adjacent teeth 415 and 430, respectively. As illustrated with arrow 480, the angle of virtual crown 400 with respect to the Z axis may be set so that the MD axis matches the arch tangent.

It is noted that the same kind of positioning may be extrapolated to place several virtual crowns in a row and even distally placed virtual crowns, it being observed that for distally placed virtual crowns, the benefit of using the arch is bigger, for example to follow the bone orientation that can be different from the alignment of the crowns.

It is also noted that according to some embodiments, if one of the adjacent teeth shows an axis far from orthogonal to the axis joining adjacent crown parts, the less tilted adjacent tooth may be used as the reference adjacent tooth axis.

Turning back to Figure 2 and after having positioned the virtual crown, the bone wherein the implant is to be implanted is segmented, for example using the CBCT 3D volume data (step 250). Such a segmentation may be done locally, in the implant site surroundings. It can be based on an analytic approach (e.g. based on watershed) or on a 3D DL algorithm in local 3D patches. Again, the 3D DL algorithm may be trained with training data comprising, as input data, a set of voxels, such as a cube of voxels belonging to CBCT 3D volume data (e.g., a 3 centimetre cube), and comprising, as output data, the segmented bone of the set of voxels (or cube). The 3D DL algorithm may also be based on the 3D Unet.

According to some embodiments, the inferior alveolar nerve canal within the mandibular jaw bone may be detected and localized, for example using Al driven automatic mandibular nerve canal tracing (e.g., using the nerve canal tracing function of the CS 3D Imaging software developed by Carestream Dental LLC, for example in its release 3.10.43 of January 15, 2024). Such a detection may be based on the previously determined mandibular arch. It may be a strict CBCT 3D volume segmentation according to which a binary value is assigned to each voxel to indicate whether it belongs to the nerve canal or a line segmentation according to which points centered on the nerve canal are determined to be depicted on coronal views or on views orthogonal to the arch. Such a detection may be based on DL approaches.

In addition to segmenting the bone, other anatomical features may be detected and/or segmented depending on the implant site location. For the sake of illustration, such anatomical features may include one or more of the following:
- the anterior loop and the extension of the mandibular canal;
- the mental foramen (or mental nerve);
- the maxillary sinus / floor;
- the nasal cavity / floor; and/or
- the incisive nasopalatine canal / nerve.

If pathologic features, such as an abscess, a cyst, and/or a tumor, are detected, they may also be segmented and possibly taken into consideration for selecting and/or positioning the implant and/or the virtual crown.

Bone quality and/or density may also be taken into account. Each of the quality and density may be scored separately or in combination. Bone volume portions could be segmented according to a given threshold (e.g., a predetermined threshold) based on the score and taken into account for positioning the implant.

An example of bone density assessment is described in patent application no. WO2019040932.

Next, a type of implant is selected (step 255). This may comprise choosing a brand and a model. This step may be carried out by the practitioner, to give him or her the possibility to select a type of implant he or she knows and/or he or she is used to using. According to some embodiments, features of existing implants of the selected type are obtained automatically, for example the existing lengths, diameters, etc., so that one or more implants of the selected type may be identified as a function of constraints of the implant site (e.g., the proximity of the nerve canal and of the bone walls).

After having selected a type of implant, some features of the implant to be used are determined and an implant and a position are identified (step 260).

For the sake of illustration, the diameter and the length of the implant to be used may be determined as a function of the virtual crown, of the segmented bone, of the nerve canal location, etc.

The implant features, as well as the position of the implant, should comply with certain rules and/or recommendations and, thus, are subject to compromises. The aim of determining the features of the implant is to make it possible to identify an implant that complies with clinical minimal safety margins, that is compatible with the virtual crown, and that may be positioned in an optimal position. The rules and/or recommendations to follow may include one or more of the following:
- keeping security margins
   - between the implant and the bone walls (e.g., 1.5 mm),
   - between the implant and the mandibular nerve canal (e.g., 2 mm), and
   - between the implant and the roots of the adjacent teeth (e.g., 3 mm),
- being compatible with the virtual crown, which implies to define a range of possible or "admissible" implant positions according to the virtual crown, and
- finding an optimal position, which requires to define "good" positioning characteristics.

According to some embodiments, identifying an implant to be used is carried out in three steps:
- determining a set of possible positions for the implant, relative to the virtual crown and the segmented bone,
- listing the implants that comply with the margins for a given one of the possible positions, which may be done by testing several implant dimensions, and
- identifying the optimal implant among the listed ones and the optimal position.

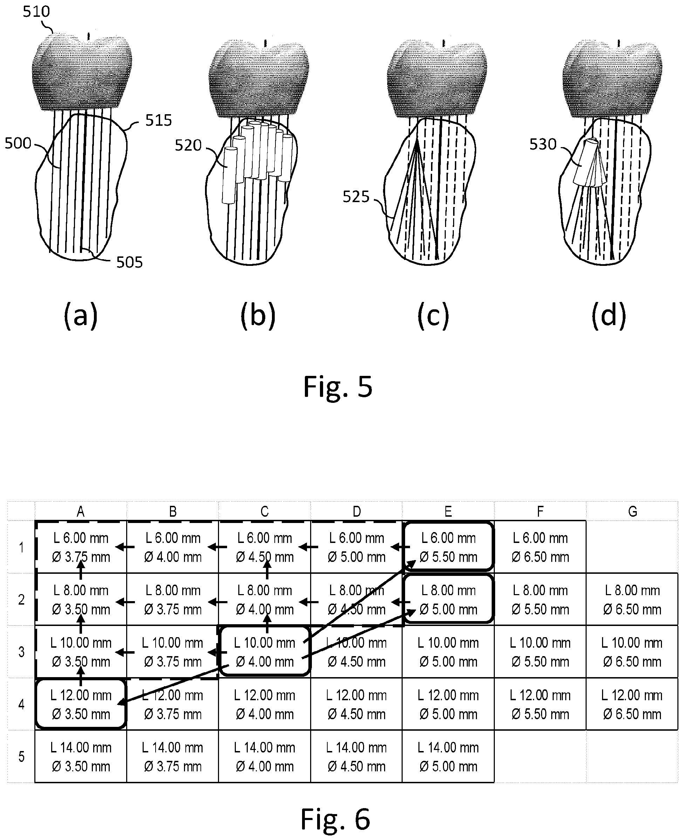

**Figures 5a** to **5d** illustrate an example of a method for determining a set of possible positions for an implant.

As illustrated in Figure 5a, a first sub-step is directed to defining a set of straight lines, generically referenced 500, for example parallel to the axis 505 of the virtual crown 510, extending into the bones 515, along the VL axis, for example based on the crown width. The maximum shift, corresponding to the crown width in the Vestibule Lingual axis minus the implant width, between crown center and implant axis depends on the size of the virtual crown. In some embodiments, the order of magnitude is -2.5 mm to +2.5 mm, every 0.1 or 0.2 mm, preferably every 0.1 mm.

Next, a position of an implant is determined, for each of the straight lines, by simulating burying a virtual implant (e.g., implant 520) in the bone, so that one end of the implant is flush with the surface of the bone, as illustrated in Figure 5b. Next, for each of these positions, a set of axes is determined by varying the angle of the virtual implant, for example by rotating the virtual implant around its end that is flush with the surface of the bone, according to an axis parallel to the MD axis (i.e., by varying the torque angle), as illustrated in Figure 5c (e.g., axis 525 that corresponds to axis 500 in Figure 5a). For example, the angle may vary from -10° to +10°, with 2 degrees steps. Next, a position of the implant is determined, for each of these new axes, by simulating burying a virtual implant (e.g., implant 530) in the bone, so that one end of the implant is flush with the surface of the bone, as illustrated in Figure 5d.

According to some embodiments, the angle between the axis of the implant and the axis of the crown is less than 10 degrees, or less than 12 degrees at the most.

After having determined the set of possible positions of the implant, the second step aims at determining the implant features, in particular the implant dimensions, that comply with the clinical margins for the selected type of implant. To that end, implants of the selected type may be virtually tested, for each of the determined possible positions, for example to determine the implant having the largest dimensions that meet the clinical margins. According to some embodiments, an implant of the selected type having preferred dimensions (e.g., a length of 10 mm and a diameter of 4 mm) is tested first, for a first possible position. Such preferred dimensions may be defined by default or chosen by the practitioner. If this implant complies with the clinical margins, it is selected. On the contrary, if the tested implant does not comply with one of the clinical margins, other implant dimensions of the selected type are tested and the process is repeated until at least one implant is selected.

According to some embodiments, when an implant does not comply with dimension requirements, a next implant to be tested is an implant having iso-surface implant dimensions with regard to the one previously tested. If all the implants, of the selected type, having iso-surface implant dimensions have been tested, and none of them complies with the dimension requirements, an implant having strictly shorter or thinner implant dimensions is tested.

According to some embodiments, the process is repeated for selecting several implants.

Once one or more implants complying with the requirements have been identified, the process is repeated with another position (if any).

Figure 6 illustrates an example of a method for selecting implants having dimensions that comply with clinical margins.

In this example, it is assumed that the preferred dimensions are those given in the C3 cell.

A test is carried out to determine whether the dimensions of the considered implant comply with the clinical margins. If the dimensions of the considered implant comply with the clinical margins, the implant is selected. On the contrary, if the dimensions of the considered implant do not comply with the clinical margins, it is determined whether there is one or more implants with iso-surface implant dimensions. In the illustrated example, the implants having the dimensions given in cells A4, E1, and E2 have iso-surface implant dimensions. According to some embodiments, if several implants have iso-surface implant dimensions, they are ordered according to their length, a priority being given to the longer length, and tested one after the other. If the dimensions of one of these implants, considered in their order, comply with the clinical margins, the corresponding implant is selected.

On the contrary, if none of the implants having iso-surface implant dimensions complies with the clinical margins, the implants having the closest smaller dimensions to the ones of the previously considered implants are considered. Therefore, according to the example illustrated in Figure 6, the implants having the dimensions given in cells D1 (identified from previous considered implant E1), D2 (identified from previous considered implant E2), C2 and B3 (identified from previous considered implant C3), and A3 (identified from previous considered implant A4) are tested. Again, according to some embodiments, if there are several implants to consider, they are ordered according to their length, a priority being given to the longer length, and tested one after the other. If the dimensions of one of these implants, considered in their order, comply with the clinical margins, the corresponding implant is selected.

The process is repeated until at least one implant is selected.

Next, after having selected implants that fit the possible positions, an optimal implant and position are identified as a tradeoff between different constraints, in particular with regard to the relative position of the implant in relation to the virtual crown, its dimensions, and the anatomical features of the implant site. To that end, a score may be assigned to each implant and position, each score being computed according to common criteria. A first criterion is the relative position of the implant in relation to the virtual crown: the closer the implant axis to the virtual crown axis (angle and/or centering on the virtual crown occlusal center), the higher the score. A second criterion may be directed to the distance between the implant and the clinical margins. Still another criterion may be the distance to the bone crest (i.e., the part of the bone that is close to the virtual crown). Of course, this list is not exhaustive, and any other clinically relevant measures could be used. In addition, each criterion may be weighted.

The optimal implant and position may be the one having the best score or one having a score that is close to the best score.

Still according to some embodiments, the implants and positions having the best scores may be provided to the practitioner who select the one that are to be used.

Naturally, other methods may be used to identify an implant to be used and/or to determine its position.

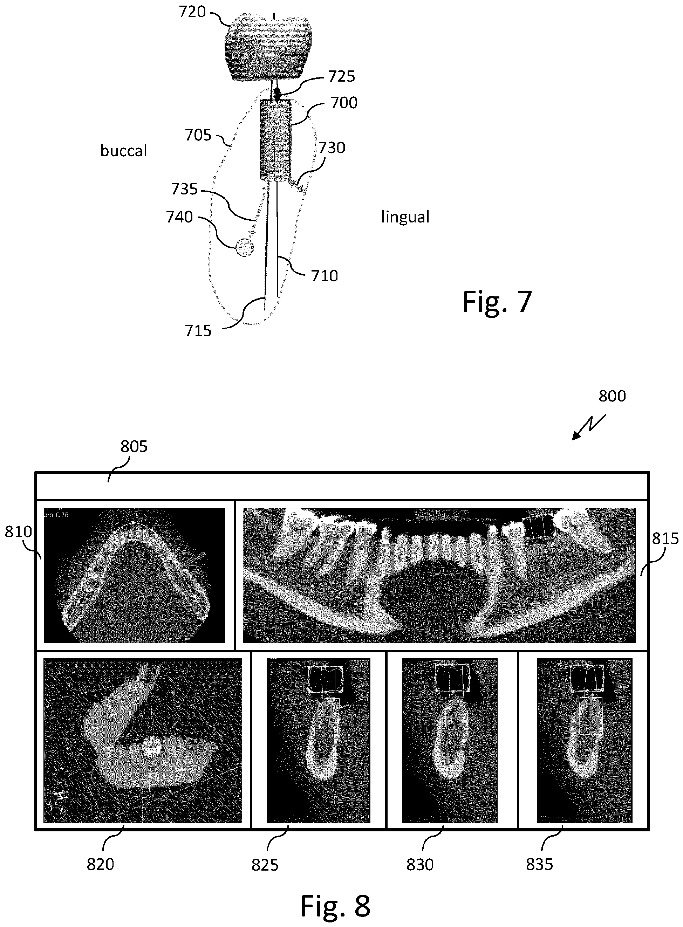

**Figure 7** illustrates an example of an implant to be used and of its position.

As illustrated, implant 700 is positioned in bone 705 so that its axis 710 is close to axis 715 of virtual crown 720, distance 725 to the bone crest is 0.9 mm, distance 730 to the bone walls is 2.4 mm, and distance 735 to nerve canal 740 is 8.9 mm.

Turning back to Figure 2 and after having identified the implant and the position to be used, the implant to be used is displayed at the identified position (step 265), enabling the practitioner to check easily the correct implant planning position (it being noted that because the implant is automatically placed, its coordinates are known and can be used to display the appropriate views).

According to some embodiments, one or several views of the implant site and of the implant are displayed, for example one or several of the followings:
- a pseudo panoramic view along the detected arch, enabling the practitioner to check if the implant axis is parallel to the at least one adjacent tooth,
- a cross sectional view passing through the implant, making it possible to check implant position regarding the anatomical features or pathological features,
- a 3D rendering view, and/or
- a view including the implant axis.

Optionally, the practitioner may shift the position of the cross-sectional view along the arch in order to screen the implant site with the implant position. Still optionally, the practitioner may display a cross-sectional view passing through the implant and screen the parallel views to the cross-sectional view.

Other views (e.g., axial or coronal view) may also be used with an appropriate screening. Each of these views may be automatically generated as well as their corresponding screening.

Optionally, the system may provide one view including the implant axis. The practitioner may be able to rotate the view around the implant axis and check if the implant position is correct.

Still optionally, the system provides one view perpendicular to the implant axis. The practitioner may be able to move the view along the implant axis to check the implant position.

Still optionally, the rendered views may be enhanced with measures, data margin constraints, etc. These items of data may be represented by numerical values and/or as graphical representations. For the sake of illustration, margin constraints may be represented as a cylinder around the implant axis.

Displaying useful data for the practitioner helps him/her to validate the position and orientation of the implant. For example, such data may comprise one, several, or all the following items of data:
- the (shortest) distance from the implant to an external surface of the bone,
- the (shortest) distance from the implant to an anatomical feature in the site and/or in the neighborhood of the site,
- the angle of the implant with respect to the Z axis in the Z-MD plane,
- the angle of the implant with respect to the Z axis in the Z-VL plane,
- the arch path or trace,
- the representations of one or more anatomical features, for example a representation of the inferior alveolar nerve canal.
- etc.

According to some embodiments, the practitioner may modify the position and/or orientation of the implant (270), for example through the graphical user interface used to display the implant in the implant site, for example using a mouse and/or a touch screen for modifying the position and/or orientation of the implant on the right view.

As illustrated with the loop on step 265, display of the implant is automatically updated, for the one or several views, as soon as its position and/or orientation is modified.

**Figure 8** illustrates an example of a graphical user interface that may be used to display and manipulate an implant in an implant site, the implant being initially automatically selected and positioned.

As illustrated, graphical user interface 800 comprises several areas among which menu area 805, making it possible for the practitioner to select function (e.g., for accessing stored data, saving data, controlling a scanner for acquiring data, etc.), options (e.g., display options, scale of view, etc.), etc.

In addition, graphical user interface 800 comprises several display areas, here 6 display areas. According to the illustrated example, the display areas are the following,
- area 810 representing an axial view of the arch;
- area 815 representing a pseudo-panoramic view along the arch;
- area 820 being a 3D rendering view comprising the virtual crown;
- area 825 being a cross-sectional view in a mesial plan parallel to a plan perpendicular to the arch and comprising the implant;
- area 830 being a cross-sectional view according to the plan perpendicular to the arch and comprising the implant; and
- area 835 being a cross-sectional view in a distal plan parallel to the plan perpendicular to the arch and comprising the implant.

Rendering these items of information visible simultaneously (or a subset of these items of information or equivalent items of information) makes it possible to reduce opportunity for error by failing to check some of the requirements such as clinical margins.

According to some embodiments, the practitioner may modify the position and/or the orientation of the implant in anyone of the views, all the views being dynamically updated as a function of the modifications.

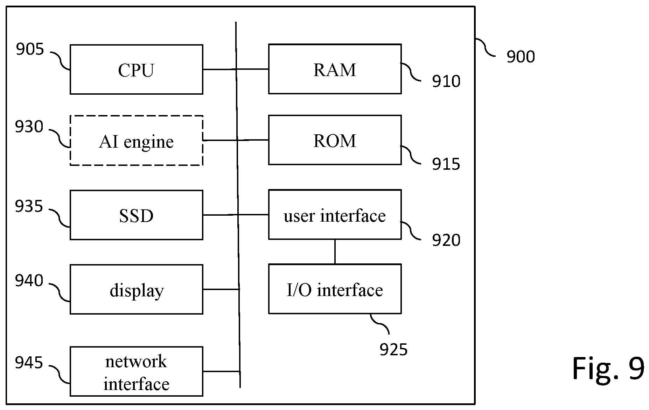

**Figure 9** is a schematic block diagram of computer for implementation of one or more embodiments of the disclosure, in particular for carrying out the steps or parts of the steps described by reference to Figure 2.
computer 900 comprises a communication bus that may be connected to all or some of the following elements:
- a central processing unit 905, such as a microprocessor, denoted CPU;
- a random access memory 910, denoted RAM, for storing the executable code of the method of some embodiments of the disclosure as well as the registers adapted to record variables and parameters necessary for implementing a method for carrying out dental implant planning according to some embodiments of the disclosure, the memory capacity of which can be expanded by an optional RAM connected to an expansion port for example;
- a read-only memory 915, denoted ROM, for storing computer programs for implementing some embodiments of the disclosure;
- a user interface 920 which can be used for receiving inputs from a user and providing information to a user;
- an input/output interface 925, for example for receiving data from scanners (e.g., from a CBXT scanner and/or a intraoral scanner) and/or for transmitting data to external devices, in signal communication with computing device 900 via wires or via a wireless link; and
- a display 540 for displaying information in relation with dental implant positioning.

Optionally, the communication bus of computing device 500 may be connected to a solid-state disk 935 denoted SSD (or a hard disk) used as a mass storage device and/or to an Al engine 930 which may configured for analyzing data, for example to segment teeth, bones, etc. It is noted that the AI engine may be embedded within computing device 900, may be in a remote processing unit, or may be spread over one or more servers, for example in a cloud.

The communication bus of the computer 900 may also be connected to a network interface 945 typically in signal communication with a communication network over which digital data can be transmitted or received for receiving/sending data from/to remote devices, in particular to a dental information system and/or storage device 935. The network interface 945 can be a single network interface, or composed of a set of different network interfaces (for instance wired and wireless interfaces, or different kinds of wired or wireless interfaces). Data packets are written to the network interface for transmission or are read from the network interface for reception under the control of the software application running in the CPU 905

The executable code may be stored either in read-only memory 915, on solid state device 935 or on a removable digital medium such as for example a memory card. According to a variant, the executable code of the programs can be received by means of a communication network, via the network interface 945, in order to be stored in one of the storage means of the computing device 900, such as solid-state device 935, before being executed.

Central Processing Unit (CPU) 905 is adapted to control and direct the execution of the instructions or portions of software code of the program or programs according to some embodiments of the disclosure, the instructions being stored in one of the aforementioned storage means. After powering on, CPU 905 is capable of executing instructions from main RAM memory 910 relating to a software application after those instructions have been loaded from ROM 915 or from solid-state device 935 for example. Such a software application, when executed by CPU 905, causes the steps herein disclosed to be performed.

Any step herein disclosed may be implemented in software by execution of a set of instructions or program by a programmable computing machine, such as a PC ("Personal Computer"), a DSP ("Digital Signal Processor") or a microcontroller; or else implemented in hardware by a machine or a dedicated component, such as an FPGA ("Field-Programmable Gate Array") or an ASIC ("Application-Specific Integrated Circuit").

Although the present disclosure has been described herein above with reference to some specific embodiments, the present disclosure is not limited to these specific embodiments, and modifications will be apparent to a person skilled in the art which lie within the scope of the present disclosure.

Many further modifications and variations will suggest themselves to those versed in the art upon making reference to the foregoing illustrative embodiments, which are given by way of example only and which are not intended to limit the scope of the disclosure, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

## Claims

1. A computer-assisted method for assisting a practitioner for planning a dental implant, the method comprising:
obtaining (200, 205) a 3D representation of at least a portion of a dental arch;
segmenting (235), from the 3D representation, teeth adjacent to a site of the dental implant and of an associated crown and segmenting (250), from the 3D representation, a bone comprising a portion of the site;
selecting the dental implant and determining (260) a position of the dental implant as a function of the segmented teeth and bone; and
displaying (265) at least a portion of the segmented teeth and bone and displaying the selected dental implant at the determined position.

2. The method of claim 1, further comprising segmenting, from the 3D representation, anatomical features in the site and/or in the neighborhood of the site, the selecting the dental implant and the determining the position of the dental implant being further carried out as a function of the segmented anatomical features.

3. The method of claim 1 or claim 2, wherein the 3D representation comprises at least Cone Beam Computed Tomography, CBCT, 3D volume data, the segmenting the bone comprising a portion of the site comprising segmenting the CBCT 3D volume data.

4. The method of claim 3, wherein the 3D representation further comprises at least one 3D mesh model registered with the CBCT 3D volume data, the segmenting the teeth adjacent to the site comprising segmenting the 3D mesh model.

5. The method of any one of claims 1 to 4, further comprising positioning (245) the associated crown in the site, between the segmented teeth, the dental implant being selected as a function of the segmented teeth, the segmented bone, and the positioned associated crown.

6. The method of claim 5, further comprising determining a shape of at least a portion of dental arch, the associated crown being placed as a function of the determined shape and of a position of the segmented teeth relative to the determined shape.

7. The method of claim 5 or claim 6, further comprising displaying the associated crown.

8. The method of any one of claims 5 to 7, wherein the position of the dental implant is determined as a function of the segmented teeth, the segmented bone, and the positioned associated crown.

9. The method of any one of claims 1 to 8, further comprising selecting the associated crown.

10. The method of any one of claims 1 to 9, further comprising obtaining (255) a dental implant type, the dental implant being selected as a function of the segmented teeth, the segmented bone, and the dental implant type.

11. The method of any one of claims 1 to 9, wherein selecting the dental implant and determining a position of the dental implant comprise determining a plurality of possible positions for the dental implant and selecting at least one dental implant for at least one position of the plurality of possible positions.

12. The method of any one of claims 1 to 11, wherein selecting the dental implant comprises determining features of the dental implant, the dental implant being selected as a function of the determined features.

13. The method of any one of claims 1 to 12, wherein displaying a least a portion of the segmented teeth and bone and displaying the selected dental implant at the determined position comprises displaying a least a portion of the segmented teeth and bone and displaying the selected dental implant at the determined position in a plurality of views.

14. The method of claim 13, wherein the plurality of views comprises at least one cross-sectional view according to a plan perpendicular to a Mesio-Distal axis and comprising at least a portion of the dental implant and comprises at least one cross-sectional view according to a plan perpendicular to a Vestibulo-Lingual axis and comprising at least a portion of the dental implant.

15. The method of any one of claims 1 to 14, further comprising displaying additional data, the additional data comprising at least one of a distance from the implant to an external surface of the bone, a distance from the implant to an anatomical feature in the site and/or in the neighborhood of the site, an angle of the implant with respect to a predetermined frame reference, an arch path, and/or representations of one or more anatomical features.

16. The method of any one of claims 1 to 15, further comprising obtaining an indication of a change of the position of the dental implant and updating display of the selected dental implant according to the changed position.

17. A non-transitory computer-readable storage medium comprising a sequence of instructions for implementing each of the steps of the method according to any one of claims 1 to 16 when loaded into and executed by a computer.

18. A device comprising a processing unit configured for carrying out each of the steps of the method according to any one of claims 1 to 16.

19. A computer program product for a programmable apparatus, the computer program product comprising a sequence of instructions for implementing each of the steps of the method according to any one of claims 1 to 16 when loaded into and executed by the programmable apparatus.
